# EUROPÄISCHE PATENTSCHRIFT

(11) **EP 1 725 153 B1**
(45) Veröffentlichungstag und Bekanntmachung des Hinweises auf die Patenterteilung: **13.08.2008**
(21) Anmeldenummer: 05707243.1
(22) Anmeldetag: 07.02.2005
(51) Int. Cl.: A47L 7/04, A47L 9/10, A47L 9/12, A47L 9/14, B01J 20/18, B01J 20/20, B01J 20/26, B01J 20/32

(54) **ADSORBENS, STAUBSAMMELRAUM SOWIE VERFAHREN ZUR GERUCHSADSORPTION**
ADSORBING AGENT, DUST COLLECTION CHAMBER, AND METHOD FOR THE ADSORPTION OF SMELLS
AGENT ADSORBANT, COMPARTIMENT COLLECTEUR DE POUSSIERE ET PROCEDE POUR ADSORBER DES ODEURS

(30) Priorität: 01.03.2004 DE 102004009956; 15.10.2004 EP 04024608
(43) Veröffentlichungstag der Anmeldung: 29.11.2006
(73) Patentinhaber: Eurofilters N.V., 3900 Overpelt (BE)
(72) Erfinder: SCHULTINK, Jan, Eurofilters N.V., Nolimpark 1013, B-3900 Overpelt (BE); SAUER, Ralf, Eurofilters N.V., Nolimpark 1013, B-3900 Overpelt (BE)
(74) Vertreter: Pfenning, Meinig & Partner GbR
(86) Internationale Anmeldenummer: PCT/EP2005/001214
(87) Internationale Veröffentlichungsnummer: WO 2005/082219

(56) Entgegenhaltungen:
- DE-A1- 4 204 553
- DE-A1- 19 513 658
- DE-A1- 19 650 749
- DE-U1- 9 317 809
- US-A- 6 010 550
- US-B1- 6 630 233
- PATENT ABSTRACTS OF JAPAN Bd. 011, Nr. 133 (C-418), 25. April 1987 (1987-04-25) & JP 61 271013 A (OJI SEITAI KK), 1. Dezember 1986 (1986-12-01)

## Beschreibung

Die Erfindung betrifft ein neues Staubsammelfilters mit Adsorbens, insbesondere zur Geruchsabsorption. Das Adsorbens besteht aus einem spezifischen Trägermaterial und einem Adsorptionsmaterial. Die Erfindung betrifft weiterhin einen Staubsammelraum in dem das Adsorbens enthalten ist. Letztlich betrifft die Erfindung noch ein Verfahren zur Geruchsadsorption.

Im Stand der Technik sind bereits verschiedene Maßnahmen bekannt geworden, die zu einer Reduzierung von Gerüchen aus in Filtern abgeschiedenen Stäuben führen.

Eine Lösung besteht darin, dass die mit Geruchsstoffen belastete Luft durch ein separates nachgeschaltetes Filter geführt wird. Dabei kommen Schüttbettfilter aber auch Filter aus mit Kohle oder anderen Adsorbentien beschichteten Trägerstrukturen zum Einsatz. Eine derartige Lösung ist in der GB 2 288 749 beschrieben.

Im Stand der Technik ist es auch bekannt geworden durch Einbringen von mit Duftstoffen getränkten Körpern, in den Filterraum, eine Überdeckung von Gerüchen zu erreichen. Verwendet werden hierzu mit Parfüm getränkte Fasergebilde, die mit einer Kunststoffhülle ummantelt sind, Naturstoffe wie z. B. Orangenkerne oder Orangenschalen, Kunststoffteile die während des Spritzgussvorganges mit Parfüm oder natürlichen ätherischen Ölen beaufschlagt werden aber auch anorganische Trägermaterialien wie Sand/Carbonate, die mit Duftstoffen getränkt sind, (WO 94/21305). In der US 5,461,751 A werden mit antibakteriellen und/oder fungiziden Substanzen getränkte Granulate beschrieben.

Letztlich ist es auch der WO 01/08543 A1 bekannt, dass man ein Adsorbens in loser Form in einen Staubsammelfilter einbringt. Als Adsorbens werden dabei Aktivkohle, die in Bruch/Kugelform oder auch als Fasern vorliegen kann sowie Zeolithe und poröse Polymere angesprochen.

Die Lösung nach WO 01/08543 zeigt schon eine befriedigende Reduktion der Geruchsstoffe in der Ausblasluft von Handstaubsaugern. Allerdings hat diese Lösung auch einen gravierenden Nachteil. Bezüglich der gleichmäßigen Verteilung des Adsorbens in der Filtertüte ist es wünschenswert, ein möglichst feines, leichtes Adsorbens zu verwenden. Dies hat weiterhin den Vorteil, dass die innere Oberfläche des Adsorbens auf zahlreichen relativ kurzen Wegen (Zugangsporen) verfügbar ist. Bei der hohen Feinstaubbelastung in einer Staubsaugerfiltertüte, durch die die Zugangsporen leicht verstopft werden können, kann nur so gewährleistet werden, dass die innere Oberfläche näherungsweise vollständig zur Geruchsadsorption genutzt wird.

Sehr feine Partikel bleiben allerdings nicht wie gewünscht, homogen mit dem Staub gemischt innerhalb der Filtertüte, sondern dringen durch die innersten Filterlagen und werden größtenteils in der Filtertütenwand abgeschieden. Dadurch erhöht sich unerwünschterweise der Widerstand der Filtertüte (Druckverlustanstieg) und das Adsorbens steht nicht mehr als Geruchsbinder zur Verfügung. Durch die Verwendung relativ grobkörniger Adsorbentien lässt sich dieser Druckverlustanstieg der Filtertüte vermeiden. Die angestrebte gleichmäßige Verteilung des Adsorbens in der Filtertüte wird durch das höhere Gewicht der Teilchen allerdings verschlechtert. Zusätzlich werden die Zugangsporen schnell verstopft und nur noch ein kleiner Bruchteil der inneren Oberfläche steht zur Geruchsadsorption zur Verfügung.

Ausgehend von der WO 01/08543 A1 ist es die Aufgabe der vorliegenden Erfindung, ein neuartiges Adsorbens sowie einen Staubsammelraum anzugeben, mit dem eine gegenüber dem Stand der Technik verbesserte Reduzierung der Gerüche von in Staubsammelräumen abgeschiedenen Stäuben erreicht wird und bei dem das Adsorptionsmaterial effektiv eingesetzt wird.

Die Aufgabe wird in Bezug auf das Staubsammelfilter durch die Merkmale des Patentanspruches 1, betreffend das Verfahren zur Geruchsadsorbtion durch die Merkmale des Patentanspruches 52 gelöst. Die Unteransprüche geben vorteilhafte Weiterbildungen an.

Gemäß der vorliegenden Erfindung wird somit vorgeschlagen, dass das Adsorbens des Staubsammelfilters aus Fasern, Flocken und/oder Granulat als Trägermaterial besteht, auf die ein pulverförmiges Adsorptionsmaterial aufgebracht ist. Die Anmelderin konnte zeigen, dass dann, wenn ein derartiges Adsorbens z. B. in einem Staubsammelraum verwendet wird, gegenüber dem Stand der Technik nur ein Bruchteil des Adsorptionsmaterial eingestzt werden muss. Mit dem erfindungsgemäßen Staubsammerfilter wird dabei gleichzeitig eine deutliche Reduzierung von Gerüchen von den in Staubsammelräumen abgeschiedenen Stäuben erreicht. Dies ist offensichtlich darauf zurückzuführen, dass das in Fasern und/oder Flocken vorliegende Adsorbens unter den Betriebsbedingungen, z.B. eines Staubsammelfilters im Staubsammelfilter aufgewirbelt vorliegt und sich damit homogen mit dem Staub mischt.

Der Einsatz von reinem pulverförmigen Adsorptionsmaterial mit einer vergleichbaren mittleren Korngröße wie es auf den Fasern/Flocken aufgebracht ist, ist normalerweise nicht möglich, da sonst das Filtermaterial leicht verstopft wird.

Bei dem Adsorbens nach der Erfindung kann das Adsorptionsmaterial in Abhängigkeit vom Trägermaterial und/oder der Methode des Aufbringens in einer Menge von 1 bis 100 Gew.-% vorliegen. Bei der Kombination Trägermaterial-Granulat bzw. sphärische Partikel aus makroporösen Polymeren und Adsorptionsmaterial aus makroporösen Polymeren kann je nach elektrostatischer Aufladung bis zu 100 Gew.-% Adsorptionsmaterial aufgebracht werden. In den anderen Fällen wird das Adsorptionsmaterial in einer Menge von 1 bis 40 Gew.-%, bevorzugt in einer Menge von 7 bis 25 Gew.-% aufgebracht. Das Adsorptionsmaterial in Pulverform kann dabei ganzflächig oder auch nur bereichsweise auf der Oberfläche des Trägermaterials aufgebracht sein.

Aus stofflicher Sicht sind beim Adsorptionsmaterial an und für sich alle aus dem Stand der Technik bekannten pulverförmigen Materialien einsetzbar. Besonders geeignet sind dabei Aktivkohle auf Basis von Stein-, Holz-, Bambus- oder Kokosnussschalenkohle, sauer oder basisch oder mit Silbersalzen imprägnierte Aktivkohle, funktionalisierter Kohlenstoff, hydrophobe Zeolithe und/oder hydrophobe, makroporöse Polymere.

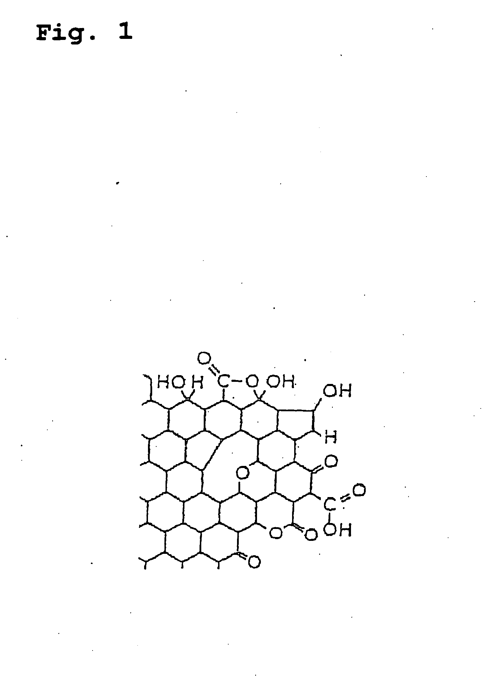

Die Anmelderin konnte zeigen, dass neben pulverförmiger Aktivkohle auch funktionalisierter Kohlenstoff in Form eines aromatischen Kohlenstoffgerüstes mit funktionellen Gruppen besonders geeignet ist. Ein derartiges Adsorbens ist unter der Bezeichnung Carbonized Basal Plates (CBP) bekannt geworden. Eine Beschreibung dieser Materialien ist bei R. Kunz, 1816 North Cascade Avenue, Colorado Springs zu finden. In Fig. 1 ist die Struktur eines derartigen Adsorbens wiedergegeben. Dieses Adsorbens hat sich als besonders geeignet erwiesen. Als weiterhin geeignet hat sich pulverisierte Bambusknoten Aktivkohle erwiesen. Ein derartiges Adsorbensmaterial ist z.B. von Aqua Air Adsorbens in DE-04509 Krostitz unter der Bezeichnung BW 200 erhältlich.

Bevorzugt ist es, wenn die Aktivkohle in einer mittleren Korngröße von 1 bis 100, bevorzugt 15 bis 50 µm eingesetzt wird.

Es hat sich gezeigt, dass neben der vorstehend beschriebenen Aktivkohle ausgewählte Zeolithe besonders geeignet sind. Wesentlich für die Eignung ist zunächst, dass die Mikroporen des Zeolithen eine ausreichende Größe haben. Erst oberhalb eines Durchmessers von 5 Å sind die Mikroporen in der Lage typische Geruchsmoleküle aufzunehmen und zu binden. Zusätzlich muss der Zeolith einen stark hydrophoben (unpolaren) Charakter haben. Erst ab einem Verhältnis von SiO₂/Al₂O₃ > 200 (Modul) ist ein Zeolith ausreichend unpolar um die Geruchsmoleküle zu binden. Besonders bevorzugt sind Zeolithe mit einem Modul > 300. Die Oberfläche überschreitet 400 m²/g. Die Partikelgröße der verwendeten Zeolithe lag bei 2 bis 30 µm. Das Gesamtporenvolumen liegt über 0,2 cm³/g, es sind aber auch Agglomerate dieser Partikel einsetzbar. In diesem Fall ist auch ein höheres Gesamtporenvolumen durch die entstehenden Makroporen realisierbar. Solche Zeolithe sind beispielsweise durch dealuminieren der Typen Y, 13C, ZSM5 und Beta zugänglich.

Neben den Zeolithen sind auch Bentonite, insbesondere "Fullers Erde" geeignet.

Bei den Zeolithen haben sich die käuflichen Typen DAY (Degussa) sowie TZB 9013 (Tricat) und DALY (Tricat) als gut geeignet erwiesen.

Als dritte, besonders vorteilhafte Gruppe sind die makroporösen (makroreticular) Polymere zu nennen. Ein typischer Vertreter ist das vernetzte SDVB (Styroldivinylbenzol). Es entsteht durch Copolymersiation von Styrol mit Divinylbenzol in Gegenwart von sog. Porogenen (Porenbildnern). Bevorzugt werden hydrophobe Varianten mit einer Oberfläche von > 600 m²/g und Mikroporen von 6 bis 20 Å sowie einem möglichst hohen Anteil Mesoporen (20 bis 500 Å) und Makroporen (> 500 Å) verwendet. Der durchschnittliche Porendurchmesser liegt zwischen 3 und 300 Å.

Vorteilhafterweise liegt die Partikelgröße zwischen 1 und 500 µm. Bevorzugt sind Partikel von 1 bis 200 µm. Das Porenvolumen solcher Produkte liegt typischerweise > 0,4 cm³/g. Käuflich erworben werden können solche makroporösen Polymere bei Rohm & Haas (Amberlite), Purolite (Makronet), Dow Chemicals (Optipore), Mitsubishi Chemical Company (Sepabeads) und Bayer (I-ONAC).

Geeignet ist auch eine Beschichtung mit porösen kristallinen metallorganischen Komplexen wie z.B. "MOF-177". Dieses Adsorbens realisiert eine extrem große Oberfläche (4500 m²/g) bei ausreichend großen Mikroporen (10 Å). Diese Kristalle sind beschrieben in Nature Bd. 427, Seiten 523 bis 527, Februar 2004. Auf dem Offenbarungsgehalt dieses Dokumentes wird Bezug genommen.

Als Trägermaterial für das erfindungsgemäße Adsorbens werden Fasern, Flocken und/oder Granulat vorgeschlagen.

Aus stofflicher Sicht können für das Trägermaterial des Adsorbens in Bezug auf die Fasern Chemiefasern und/oder Naturfasern eingesetzt werden. Bei den Chemiefasern wären cellulosische Fasern wie Viskose oder synthetische Fasern zu nennen. Beispiele für synthetische Fasern sind Fasern aus Polyolefinen, Polyester, Polyamiden, Polyacrylmethyl und/oder Polyvinylalkohol.

Beispiele für Naturfasern sind Cellulose, Holzfaserstoffe, Kapok, Flachs, Jute, Manilahanf, Kokos, Wolle, Baumwolle, Kenaf, Abaca, Maulbeerbast und/oder Fluffpulp.

Es hat sich weiterhin gezeigt, dass es bei den Fasern bevorzugt ist, wenn diese verzweigt, gekrimt, hohl und oder texturiert sind und/oder einen nicht kreisförmigen (z. B. trilobalen) Querschnitt aufweisen.

Von den Abmessungen her ist es günstig, wenn die Fasern eine mittlere Länge zwischen 0,3 mm und 100 mm, bevorzugt zwischen 0,5 und 70 mm aufweisen.

Die synthetischen Fasern können auch antibakteriell ausgerüstet sein. Dies kann dadurch erfolgen, dass bereits bei der Herstellung antibakterielle Stoffe zugesetzt werden. Der Vorteil dieser Fasern besteht darin, dass die antibakteriellen Stoffe praktisch nicht freigesetzt werden und keine Minderung der antibakteriellen Wirkung eintritt. Solche Fasern sind erhältlich bei Rhovyl in F-55310 Tronville en Barrois, z.B. die Fasern Rhovyl'A.S.+^{®} oder bei Japan Exlan Co. Ltd., Tokyo sowie bei Sterling Fibers Inc., 5005 Sterling Way, Pace, Fla unter dem Markennamen "biofresh" und DAK Americas, 5925 Carnegie Blvd., Charlotte, NC 28209.

Selbstverständlich ist es auch möglich, die Fasern nachträglich antibakteriell auszurüsten.

Gemäß der vorliegenden Erfindung ist es weiterhin vorgesehen, dass als Trägermaterial nicht nur Fasern eingesetzt werden, sondern auch Flocken. Als geeignete Materialien wären hierbei zu nennen Schaumstoffe, Vliesstoffe, Textilien, geschäumte Stärke, geschäumte Polyolefine sowie Folien.

Bei den Flocken sind Durchmesser von 0,3 bis 30 mm, bevorzugt 0,5 bis 20 mm vorteilhaft. Besonders günstig ist ein Durchmesser von 1 bis 9,5 mm.

Erfindungsgemäß kann als Trägermaterial auch ein Granulat eingesetzt werden. Als Granulat im Sinne der Erfindung werden auch sphärische Partikel verstanden. Derartige sphärische Partikel aus Polymeren werden in der Technik als "Beads" bezeichnet. Als Granulate/Beads werden dabei nach einer bevorzugten Variante insbesondere makroporöse synthetische Polymere eingesetzt. Der Vorteil dieser Variante ist darin zu sehen, dass das Adsorbens zum einen als Trägermaterial aus einem Adsorbens besteht, nämlich aus makroporösen Polymeren, auf das dann ein zweites Adsorbensmaterial in Pulverform aufgebracht wird. Beim Adsorbensmaterial können dabei alle Adsorbensmaterialien wie vorstehend bereits beschrieben, eingesetzt werden. Bei den makroporösen Polymeren, die als Trägermaterial eingesetzt werden, sind solche bevorzugt, die eine Partikelgröße von 0,2 bis 1,5 mm, bevorzugt von 0,3 bis 1 zum, aufweisen. Die makroporösen Polymere als Trägermaterial können aus Polymeren bestehen, wie bereits vorstehend bei den Adsorptionsmaterialien beschrieben, bevorzugt sind diese makroporösen synthetischen Polymere aus Polystyrol, Acrylsäure und/oder deren Derivaten aufgebaut. Die spezifische Oberfläche liegt oberhalb von 300 m²/g, bevorzugt in Bereichen zwischen 400 bis 1200 m²/g. Ein weiteres Kennzeichen der porösen Polymere als Trägermaterial ist, dass diese ein Porenvolumen aufweisen, das größer ist als 0,4 ml/ml.

Derartige Trägermaterialien sind z.B. käuflich erhältlich bei der Firma Rohm & Haas und werden unter dem Markennamen Amberlite XAD vertrieben. Ein weiterer Anbieter ist die Firma Purolite, die unter dem Markennamen Makronet MN ebenfalls geeignete Polymere anbietet.

Das erfindungsgemäße Adsorbens ist dabei so aufgebaut, dass auf dem Trägermaterial wie vorstehend beschrieben, das pulverförmige Adsorbtionsmaterial chemisch und/oder physikalische auf die Oberfläche des Trägermaterials aufgebracht ist.

Ein Aufbringen im Sinne der vorliegende Erfindung kann so erfolgen, dass erwärmtes Adsorptionsmaterial auf die Oberflächen des Trägermaterials aufgebracht wird, so dass durch Wärmeübertragung auf die Oberfläche des Trägermaterials ein Anschmelzen erfolgt und die pulverförmigen Partikel haften. Andererseits kann auch die Oberfläche des Trägermaterials erweicht werden und dann die Partikel auf deren Oberfläche aufgebracht werden. Bei Bikomponentenfasern ist es möglich, dass die äußere Schicht einen niedrigeren Schmelzpunkt als der Kern aufweist, so dass durch dessen Erwärmung eine Haftung der Partikel möglich ist.

Physikalisch kann das Aufbringen dadurch erfolgen, dass elektrostatisch geladenes Trägermaterial eingesetzt wird. Es kann mit triboelektrisch oder mit durch Coronaladung aufgeladenem Trägermaterial gearbeitet werden. Bevorzugt werden z.B. geladene Splitfasern verwendet. Es ist auch möglich, die Haftung des Adsorptionsmaterials an dem Trägermaterial dadurch zu erzielen, dass geeignete Fasern, Flocken, Granulate und/oder Beads und Adsorptionsmaterial gemischt werden, wobei durch den triboelektrischen Effekt das Trägermaterial und die Adsorbtionsteilchen entgegengesetzt aufgeladen werden. Man erreicht so eine hervorragende elektrostatische Bindung der Adsorbensteilchen an das Trägermaterial, ohne die Oberfläche der Adsorbensteilchen durch Bindemittel zu reduzieren. Besonders vorteilhaft lässt sich diese Möglichkeit bei der Variante synthetische Fasern mit makroporösen Polymeren einsetzen. So zeigt beispielsweise die Kombination Polypropylen-Fasern mit SDVB (Styroldivinylbenzol) Pulver beim Mischen eine starke triboelektrische Aufladung. Es hat sich überraschend herausgestellt, dass bei der Kombination von einem makroporösen SDVB als Trägermaterial mit einem Adsorbtionsmaterial auf Basis von mit Amin funktionalisiertem makroporösem SDVB ein stark ausgeprägter triboelektrischer Effekt auftritt, der zu einer besonders festen Anbindung des Adsorptionsmaterials an den Träger führt.

Das Adsorbens wie vorstehend beschrieben, kann auch in einer luftdurchlässigen Umhüllung vorliegen. Der Vorteil dieser Ausführungsform ist darin zu sehen, dass das Adsorbens leicht handhabbar ist und dann, wenn es z.B. in einem Staubsammelfilter in einem Staubsauger eingesetzt wird, problemlos in den Staubsammelfilter eingebracht werden kann. Die Umhüllung für einen derartigen Anwendungsfall ist dabei so aufgebaut, dass sie unter den Betriebsbedingungen wieder zerstört wird, so dass das Adsorbens im Staubsammelfilter aufgewirbelt und in Zirkulation gehalten werden kann. Geeignete Materialien sind hierfür Vliese, z. B. Nonwoven mit geringer Grammatur z. B. Meltblown mit 5 gr/m².

Ein Staubsammelraum mit einem Staubsammelfilter nach der Erfindung zeichnet sich nun dadurch aus, dass ein Adsorbens wie vorstehend beschrieben, enthalten ist. Es hat sich als günstig herausgestellt, wenn im Staubsammelraum pro 1000 cm³ Volumen 0,03 bis 5 g des Adsorbens enthalten sind. Besonders bevorzugt liegt die Menge des Adsorbens pro 1000 cm³ bei 0,3 bis 2 g Adsorbens. Bei der Ausführungsform pulverförmiges Adsorptionsmaterial auf Granulat in Form von makroporösen Polymeren sind Mengen von 0,05 bis 1 g/1000 ml ausreichend. Bei einem Staubsammelraum wie vorstehend beschrieben, handelt es sich bevorzugt um sog. beutellose Staubsauger, wie z.B. Zyklonstaubsauger.

Für Zyklonstaubsauger ist es günstig, wenn poröse Polymere als Adsorbtionsmaterialien eingesetzt werden, da dies keine zusätzliche Verschmutzung durch Kohleabrieb oder Unterkornanteil verursacht und ein Verkratzen der meist transparenten Sammelbehälter vermieden wird. Gemäß der vorliegenden Erfindung wird unter einem Staubsammelraum auch ein Abfallsammelbehälter, z.B. Müllbeutel, verstanden.

Gemäß der vorliegenden Erfindung werden unter einem Staubsammelraum insbesondere auch solche verstanden, bei denen der Staubsammelraum durch einen Staubsammelfilter gebildet wird, der aus einem von Luft durchströmbaren Filtermaterial besteht. Um eine optimale Wirkung des Adsorbens im Staubsammelfilter zu erreichen, ist es erfindungsgemäß dieses von vorne herein in loser Form in den Staubsammelfilter eingefüllt wird oder aber, dass das Adsorbens in einem in einer luftdurchlässigen Umhüllung aufweisenden Beutel im Staubsammelfilter vorliegt. Der Beutel kann dann an einer Stelle z. B. direkt im Auftreffbereich der Strömung fixiert sein. Das Adsorbens kann auch in loser Form in einem Teilbereich der Tüteninnenfläche liegen und von einer dünnen luftdurchlässigen Vliesschicht abgedeckt sein (Tasche). Dieser Bereich kann auch als durchgehender Streifen ausgebildet sein. Das Adsorbens kann auch in einem Kissen vorliegen. Hierbei wird ein Kissen eingesetzt, das aus mindestens einer Lage eines Filterpapiers oder eines speziellen Vliesstoffes besteht, auf dem das Adsorbens liegt. Das Adsorbens ist dabei dann mit mindestens einer Lage eines Vliesstoffes abgedeckt. Dieser Vliesstoff ist dabei so ausgelegt, dass unter den Betriebsbedingungen eine Zerstörung des Vliesstoffes eintritt. Selbstverständlich muss das Kissen so angeordnet sein, dass das Filterpapier/der spezielle Vliesstoff direkt auf der Innenseite der Filtertüte angebracht ist und das leichte Vlies direkt von der Luftströmung getroffen wird. Eine besonders bevorzugte Ausführungsform der luftdurchlässigen Umhüllung ist ein Kissen, das gebildet wird aus einer Lage Filterpapier mit einer Luftdurchlässigkeit > 250 1/m²/s einer Füllung mit dem erfindungsgemäßen Adsorbens und einer Lage eines Vliesstoffes mit einem Flächengewicht < 10 g/m². Dieses Kissen wird z.B. durch punktuelles Verkleben so im Staubsammelraum fixiert, dass die Papierlage des Kissens dem Filtermaterial des Staubsammelraums zugewandt ist. In diesem Zusammenhang wird der Offenbarungsgehalt der WO 2004/052500 A1, EP 1 426 090 A1 und EP1 415 699 B1 genannt.

Derartige Staubsammelfilter sind bevorzugt Staubsaugerbeutel. Diese sind dabei üblicherweise so dimensioniert und ausgelegt, dass sie mit einem Volumenstrom von 10 m³/Std. bis 400 m³/Std. durchströmbar sind. Es ist dabei bevorzugt, wenn im Staubsammelfilter pro 1000 cm³ Volumen 0,03 bis 5 g des Adsorbens enthalten sind, besonders bevorzugt 0,3 bis 2 g Adsorbens. Bei kleineren Mengen wurde festgestellt, dass dann keine ausreichende geruchsreduzierende Wirkung erreicht wird und bei größeren Mengen ist nachteilig, dass dann der Staubsammelraum als solches schon mit einem zu großen Volumen an Adsorbens gefüllt ist.

Der Staubsammelfilter nach der Erfindung besteht aus stofflicher Sicht dabei bevorzugt aus einem Filtermaterial, das ein ein- oder mehrschichtiges Papier und/oder Vliesmaterial sein kann. Derartige Filtermaterialien sind z. B. für Staubsaugerbeutel bekannt. Hierzu wird auf die EP-A 0 960 645 A1 verwiesen. Bei dem Staubsammelfilter nach der Erfindung kann es sich z. B. um einen Staubsaugerbeutel oder aber auch um einen plissierten Filter oder einen Taschenfilter handeln.

Letztlich betrifft die Erfindung ein Verfahren zur Geruchsadsorption in einen Staubsammelraum (Patentansprüche 52 bis 57).

Das erfindungsgemäße Verfahren zur Geruchsadsorption zeichnet sich dadurch aus, dass ein Adsorbens wie vorstehend beschrieben, eingesetzt wird. Bevorzugt wird dabei im Staubsammelraum mit 0,03 bis 5 g Adsorbens pro 1000 cm³ gearbeitet.

Beim erfindungsgemäßen Verfahren wird als Staubsammelraum bevorzugt ein Staubsammelfilter aus von Luft durchströmbarem Filtermaterial eingesetzt. Beim Verfahren ist dabei wichtig, dass das Adsorbens während des Betriebes des Staubsammelfilters lose im Staubsammelfilter vorliegt. Bevorzugt ist dabei der Staubsammelfilter ein Staubsaugerbeutel. Das Adsorbens wird somit entweder bei der Herstellung oder kurz nach der Herstellung in den Staubsammelfilter eingebracht und so ausgeliefert. Beim erstmaligen Gebrauch unter einem gegebenen Volumenstrom kommt es dann zu einer Aufwirbelung des Adsorbens im abgeschlossenen Staubsammelfilter und das Adsorbens kann seine wie vorstehend beschriebene geruchsmindernde Wirkung entfalten. Selbstverständlich ist es auch möglich, dass bei Beginn des Saugvorgangs das Adsorbens eingebracht wird, nämlich in der Weise, dass das Adsorbens aufgesaugt wird.

Das Adsorbens kann weiterhin in einer Umhüllung vorliegen und wieder wie vorstehend beschrieben, bereits von vorneherein im Staubsaugerbeutel enthalten sein oder aber das Adsorbens wird mit der Umhüllung vor Beginn des Saugvorgangs in den Staubsaugerbeutel eingebracht oder es wird direkt aufgesaugt.

Beim erfindungsgemäßen Verfahren ist es besonders günstig, dass auch nachträglich, d. h. direkt mit Beginn des Saugvorgangs noch das Adsorbens eingebracht werden kann, da dadurch auch bereits alle bisher gängigen Filtertüten in ihrer geruchsmindernden Wirkung einfach durch Aufsaugen oder Einbringen des Adsorbens vor dem erstmaligen Saugvorgang verbessert werden können. Selbstverständlich kann, wenn erforderlich, auch eine Nachdosierung erfolgen. Besonders bevorzugt handelt es sich beim erfindungsgemäßen Verfahren um ein Verfahren zum Staubsaugen mit einem Bodenstaubsauger oder einem Handstaubsauger und der Staubsammelfilter ist ein Staubsaugerbeutel.

Die Erfindung wird nachfolgend anhand eines Beispieles und der Fign. 1 bis 4 näher erläutert.
- Fig. 1: zeigt den Aufbau von funktionalisiertem Kohlenstoff wie er bevorzugt als Adsorbtionsmittel eingesetzt wird.
- Fig. 2: zeigt graphisch den Verlauf der Geruchsstoffkonzentration der Abluft bei verschiedenen Proben.
- Fig. 3: zeigt eine tabellarische Zusammenstellung
- Fig. 4: zeigt in photographischer Darstellung ein erfindungsgemäßes Adsorbens.

### Beispiel:

Im Folgenden werden anhand von Beispielen die durchgeführten Versuche beschrieben.

### 1) Messvorbereitung:

Die Bodenstaubsauger, Typ Miele S512-1 wurden vor der Messreihe mehrere Stunden lang mit einer leeren Filtertüte betrieben, um einen evtl. vorhandenen Geruch des Aggregats zu minimieren. Einen Tag vor dem eigentlichen Messbeginn wurde in jedes der Aggregate eine Filtertüte eingesetzt. Danach wurde jedes Aggregat komplett abgeklebt, um so ein Abblasen der Abluft durch eine andere Öffnung als die gebohrte Probennahmeöffnung (Durchmesser 13 mm) zu verhindern. Danach wurden die Aggregate in den auf 20° C eingestellten Wärmeschrank eingestellt. Weiterhin wurde die für die gesamte Messreihe nötige Menge Kaffee aus einem 500 g Vakuumpack entnommen, abgewogen und in 5 g Portionen eingeschweißt.

### 2. Versuchsablauf:

Die Versuchsfläche zum Aufsaugen des Kaffee/Staubgemisches besteht aus einer Platte Laminat mit der Grundfläche 1,21 m x 1,85 m, entsprechend 2,24 m². Die Bodenstaubsauger wurden mit der Bürsteneinstellung "Teppich" betrieben. Zur Untersuchung der Geruchsminderung im Staubsaugerbeutel wurden für jedes Aggregat an den Untersuchungstagen 1 bis 6 50g Teststaub Typ 8 (DMT, Zusammensetzung: 70 % Mineralstaub, 20 % Arbocell, 10 % Linters) sowie 5 g Kaffee (10 % Kaffee in Bezug auf die Staubmenge) gleichmäßig auf der Versuchsfläche verteilt. Am 7. und 8. Versuchstag wurden je 100g Staub und 10 g Kaffee verteilt, am letzten Versuchstag wurde nur noch beprobt. Nach dem Verteilen des Kaffee/Staubgemisches wurde der Saugfuß auf ein sauberes Stück der Versuchsfläche aufgesetzt und bei einer geringen Saugleistung (300 Watt) wurde ein Probenbeutel mit einer Gesamtlänge von 1,5 m (Füllmenge ca. 15 Liter) direkt aus der Probenahmeöffnung der abgeklebten Bodenstaubsauger befüllt. Nach dem Abnehmen der Probenbeutel (bei vollständiger Füllung) und Ausschalten des Aggregates wurde die Abklebung komplett entfernt und die Saugleistung auf maximale Leistung (1.600 Watt) hochgeregelt. Dann wurde über eine Zeitdauer von zwei Minuten das Kaffee/Staubgernisch von der Versuchsfläche abgesaugt. Nach der Probennahme wurden die Aggregate abgeschaltet, wieder komplett abgeklebt und bis zur nächsten Beprobung im Wärmeschrank gelagert. Es wurde an jedem Versuchstag Temperatur und Luftfeuchte bei der Entnahme aus dem Wärmeschrank sowie bei der Probennahme erfasst.

### 3) Untersuchte Varianten und Ausstattung der Aggregate

Alle Aggregate wurden mit Motorschutzfilter betrieben.
Variante A
   Aggregat A, 3 g Splitfaser/MN 200 MR 4636 lose in der Filtertüte,
Variante B
   Aggregat B, 3 g Splitfaser/DALY lose in der Filtertüte,
Variante C
   Aggregat C, 3 g Splitfaser/MN 200 MR 4638 lose in der Filtertüte,
Variante D
   Aggregat D, Nullvariante, leere Filtertüte,
Variante E
   Aggregat E, 3 g Splitfaser/TZB 2014 lose in der Filtertüte,
Variante F
   Aggregat F, 3 g Splitfaser/DAY lose in der Filtertüte,

### 4) Mess- und Analysenverfahren

### 4.1 Geruchsemissionen

### 4.1.1 Messverfahren; Grundlagen des Verfahrens

Bestimmung der Geruchsstoffkonzentration gemäß europäischer Norm DIN EN 13725.

### 4.1.2 Probennahmematerial

Die Probenluft wird bei der statischen Probennahme in einen Folienbeutel gezogen. Als Probenbeutel werden handelsübliche Folienschläuche verwendet, die aus geruchsfreiem Material (Nalophan NA^{©}) bestehen, welches einerseits nahezu gasdicht ist und andererseits praktisch keine Geruchsstoffe adsorbiert.

### 4.1.3 Olfaktometer

Die Olfaktometrie stellt eine kontrollierte Darbietung von mit Geruchsstoffen beladener Luft sowie eine Erfassung der dadurch beim Menschen auftretenden Sinnesempfindungen dar. Mit dem Olfaktometer wird eine Gasprobe (Geruchsstoffprobe) mit Neutralluft verdünnt und Testpersonen (Probanden) als Riechprobe dargeboten. Ein Probandenkollektiv besteht aus vier Riechern sowie einem Versuchsleiter, der für die Bedienung des Olfaktometers während eines Messvorgangs zuständig ist.

Für die beschriebenen Messungen wurde ein rechnergesteuertes Olfaktometer TO9 mit vier Probandenplätzen und automatischer Auswertung verwendet. Die Messungen wurden entsprechend DIN EN 13725 durchgeführt. Das Olfaktometer wurde mit Druckluft über eine Filtergruppe mit Silicagel (Entfeuchtung), Aktivkohle (Geruchsstoffabscheidung), Wattefilter und Glasfaser-Mikrofeinfilter (Staubabscheidung) betrieben. Die Messungen wurden entsprechend den DIN EN 13725 nach der Ja-Nein-Methode durchgeführt.

Geruchsstoffmengen werden in Geruchseinheiten (GE) gemessen, wobei eine GE der Stoffmenge eines Geruchsstoffes oder eines Stoffgemisches entspricht, die - bei 20° C und 1013 hPa in 1 m³ Neutralluft verteilt - entsprechend der Definition der Geruchsschwelle bei 50 % eines Probandenkollektivs eine Geruchswahrnehmung auslöst. Die Geruchsstoffkonzentration an der Geruchsschwelle beträgt definitionsgemäß 1 GE/m³.

Analog zum Schall werden Geruchsstoffpegel bezüglich der Schwellenkonzentration von 1 GE/m³ definiert. Dabei entspricht z.B. eine Geruchsstoffkonzentration von 100 GE/m³ einem Geruchsstoffpegel von 20 dB.

### 4.1.4 Beschreibung des Probandenkollektivs

Gemessen wurde bei den olfaktometrischen Messungen mit Versuchsleiter und vier Probanden entsprechend DIN EN 13725.

### 4.1.5 Auswertung der Proben

Die olfaktometrische Messung der Proben erfolgte maximal vier Stunden nach der Probenahme.

### 4.1.6 Anzahl der Messreihen je Messtag

12 Geruchsstoffkonzentrationsmessungen, mit je drei Reihen pro Messung. Je zwei Geruchsstoffkonzentrationsmessungen mit n-Butanol.

### 4.1.7 Weitere Untersuchungen

Um die Ergebnisse zusätzlich abzusichern, wurden die Proben an allen Messtagen auf Intensität und Hedonik (Direktbeurteilung aus dem Probenbeutel in Anlehnung an die VDI Richtlinie 3882) untersucht.

Dabei wurden vom für die Versuchsreihe typischen Kaffee(Staubgeruch) abweichende Gerüche durch die Probanden charakterisiert.

Bei den Versuchsreichen wurden Raumtemperatur und - feuchte während der Probenahme an den Messtagen erfasst sowie die Temperatur und Feuchte im jeweiligen Wärmeschrank bei der Entnahme der Aggregate.

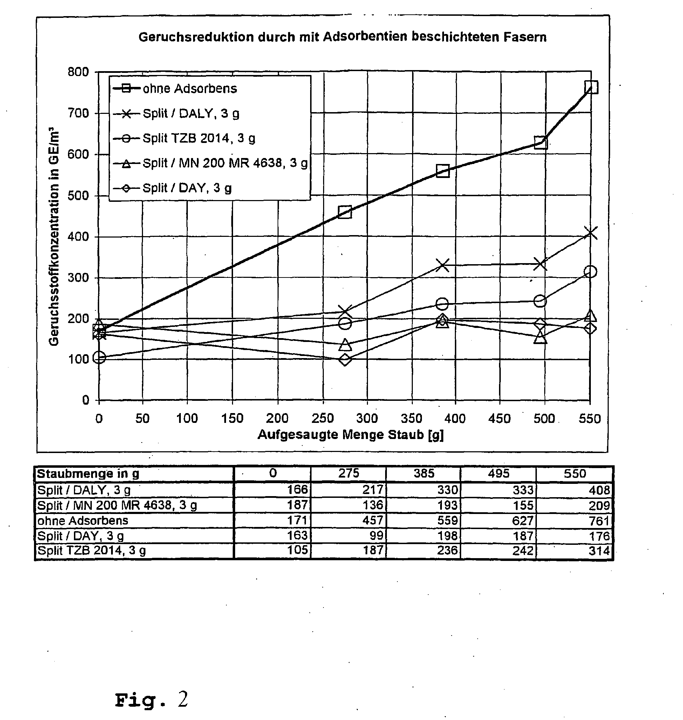

Fig. 2 zeigt den Verlauf der Geruchsstoffkonzentration der Abluft von Staubsaugern der 5 untersuchten Varianten der beschichteten Fasern im Vergleich zu einer Nullvariante. Fig. 2 macht deutlich, dass alle untersuchten Varianten eine deutliche Reduzierung der Geruchstoffkonzentration der Abluft bewirken. Überraschend ist hierbei insbesondere die gute Wirkung bei der sehr geringen Menge an Adsorbtionsmittel. Schon mit 0,3 g Adsorbtionsmittel ist eine deutliche Reduktion der Geruchsstoffkonzentration erzielbar. Die heute üblichen Lösungen verwenden dagegen 10 g Aktivkohle.

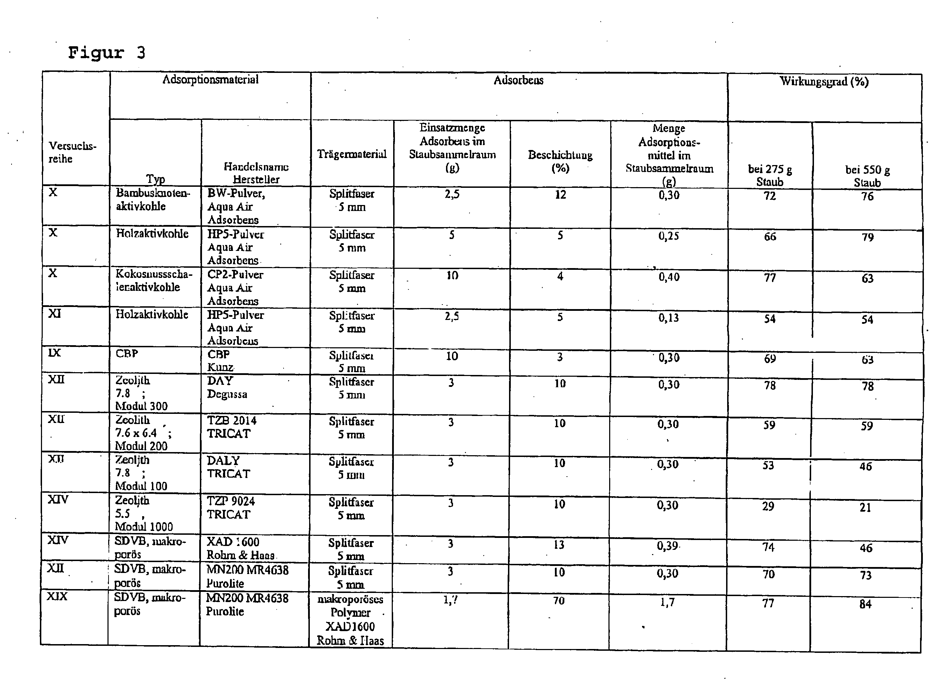

### Fig. 3 zeigt die Ergebnisse in einer tabellarischen

Zusammenstellung.

In Fig. 3 sind in der Zusammenstellung neben den Versuchen, die bereits in Fig. 2 enthalten sind und die als Versuchsreihe XII bezeichnet sind, noch Messergebnisse aus weiteren Versuchen enthalten.

Die Messergebnisse der Versuchsreihen X und XI beziehen sich dabei im Wesentlichen auf mit Aktivkohlen beschichtete Fasern. Wie aus Fig. 3 hervorgeht, zeichnet sich das erfindungsgemäße Adsorbens insbesondere dadurch aus, dass bereits bei geringsten Mengen an Adsorptionsmittel (z.B. 0,3 g Bambusknoten Aktivkohle) schon eine überdurchschnittlich hohe Minderung der Geruchsstoffkonzentration der Abluft erreicht wird.

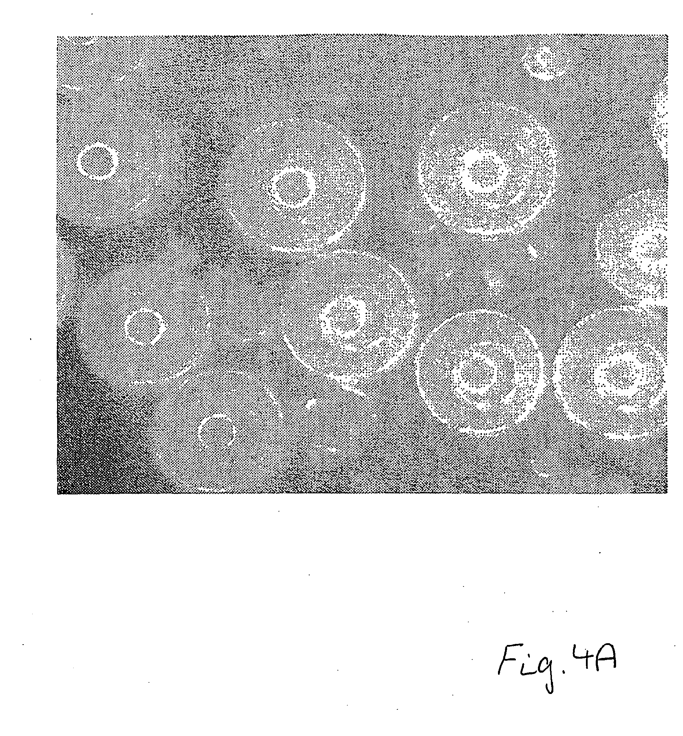

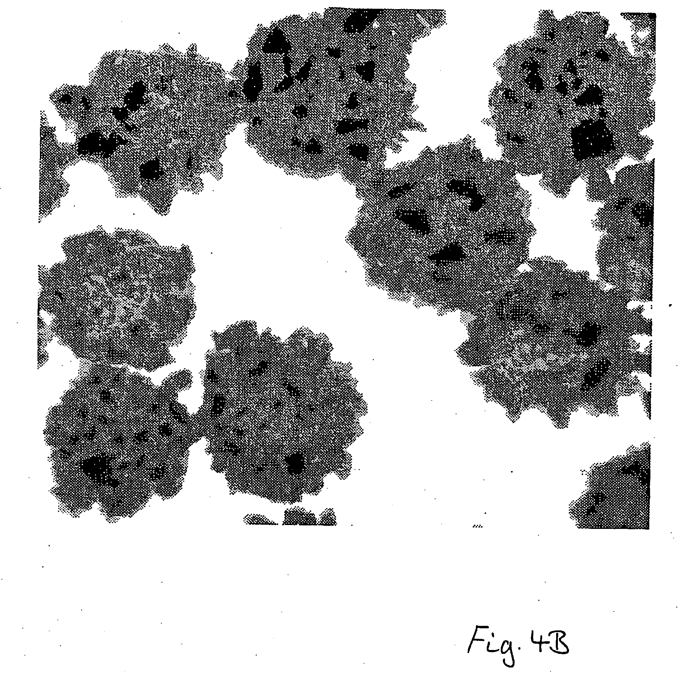

Figur 4 zeigt in Form einer photographischen Darstellung ein erfindungsgemäßes Adsorbens. Das Adsorbens, das in Figur 4 dargestellt ist, besteht aus einem makroporösen Trägermaterial (Rohm und Haas, XAD1600), das aus SDVB erhalten worden ist. Das makroporöse Trägermaterial ist in Form eines sog. "Beads" eingesetzt worden und weist einen Partikeldurchmesser von 200-350 µm auf. Beim Adsorbtionsmaterial, das auf dem makroporösen Polymer aufgebracht ist, handelt es sich um makroporöse Polymere, aus funktionalisiertem SDVB (Purolite MN200). Das Adsorptionsmaterial weist einen Partikeldurchmesser von 0-40 µm auf. Figur 4A zeigt nun unbeschichtete Beads aus XAD1600 mit einem Durchmesser zwischen 0,2 und 0,35mm. Figur 4B zeigt die erfindungsgemäßen XAD1600-Beads elektrostatisch beschichtet mit MN200-Partikeln mit einem Durchmesser zwischen 0 und 40µm. Wie Figur 4B zeigt, bedeckt das makroporöse Adsorptionsmaterial das Trägermaterial nahezu vollständig. Im Beispielsfall nach der Figur 4B wurden 70 Gew.-% Adsorptionsmaterial bezogen auf das Trägermaterial eingesetzt.

## Patentansprüche

1. Staubsammelfilter aus einem von Luft durchströmbaren Filtermaterial, wobei im Staubsammelfilter in loser Form ein Adsorbens enthalten ist,
**dadurch gekennzeichnet, dass** das Adsorbens aus Fasern, Flocken und/oder Granulat als Trägermaterial besteht, auf die oberflächlich ein pulverförmiges Adsorptionsmaterial aufgebracht ist.

2. Staubsammelfilter nach Anspruch 1, **dadurch gekennzeichnet,**
**dass** das Adsorptionsmaterial in einer Menge von 1 bis 50 Gew.-% des Trägermaterials aufgebracht ist.

3. Staubsammelfilter nach Anspruch 2, **dadurch gekennzeichnet,**
**dass** 7 bis 25 Gew.-% aufgebracht sind.

4. Staubsammelfilter nach mindestens einem der Ansprüche 1 bis 3, **dadurch gekennzeichnet,**
**dass** das Adsorptionsmaterial ausgewählt ist aus Aktivkohle, imprägnierter Aktivkohle, funktionalisiertem Kohlenstoff, hydrophobe Zeolithe, hydrophobe, poröse Polymere, Bentonite und/oder kristalline metallorganische Komplexe.

5. Staubsammelfilter nach Anspruch 4, **dadurch gekennzeichnet,**
**dass** es sich bei dem funktionalisierten Kohlenstoff um ein aromatisches Kohlenstoffgerüst mit funktionellen Gruppen handelt.

6. Staubsammelfilter nach Anspruch 4, **dadurch gekennzeichnet, dass** es sich bei der Aktivkohle um Kokosnussschalen-, Holz-, Stein- oder Bambuskohle handelt.

7. Staubsammelfilter nach Anspruch 4 oder 5, **dadurch gekennzeichnet, dass** die Aktivkohle mit sauren oder basischen Chemikalien und/oder mit Silbersalzen imprägniert ist.

8. Staubsammelfilter nach Anspruch 4, **dadurch gekennzeichnet, dass** die Zeolithe Mikroporen mit einer Porengröße > 5 Å besitzen.

9. Staubsammelfilter nach Anspruch 8, **dadurch gekennzeichnet, dass** die Porengröße der Mikroporen > 6,5 Å ist.

10. Staubsammelfilter nach Anspruch 8 oder 9, **dadurch gekennzeichnet, dass** die spezifische Oberfläche der Zeolithe > 400 m²/g ist.

11. Staubsammelfilter nach mindestens einem der Ansprüche 8 bis 10, **dadurch gekennzeichnet, dass** die Zeolithe ein Modul > 200, bevorzugt > 300 aufweisen.

12. Staubsammelfilter nach mindestens einem der Ansprüche 8 bis 11, **dadurch gekennzeichnet, dass** die Partikelgröße der Zeolithe im Bereich von 2 bis 30 µm liegt.

13. Staubsammelfilter nach Anspruch 4, **dadurch gekennzeichnet, dass** die porösen Polymere Mikroporen von 6 bis 20 Å, Mesoporen von 20 bis 500 Å und Makroporen > 500 Å aufweisen.

14. Staubsammelfilter nach Anspruch 4 oder 13, **dadurch gekennzeichnet, dass** der durchschnittliche Porendurchmesser zwischen 3 und 300 Å liegt.

15. Staubsammelfilter nach Anspruch 4, 13 oder 14, **dadurch gekennzeichnet, dass** die Partikelgröße der porösen Polymere im Bereich von 1 bis 500 µm, bevorzugt 1 bis 200 µm, liegt.

16. Staubsammelfilter nach mindestens einem der Ansprüche 4, 13 bis 15, **dadurch gekennzeichnet, dass** das Porenvolumen ≥ 0,4 cm³/g ist.

17. Staubsammelfilter nach mindestens einem der Ansprüche 4, 13 bis 15, **dadurch gekennzeichnet, dass** die porösen Polymere hydrophob sind.

18. Staubsammelfilter nach mindestens einem der Ansprüche 4, 13 bis 17, **dadurch gekennzeichnet, dass** die porösen Polymere aufgebaut sind aus Styrol, Acrylsäure und/oder deren Derivaten.

19. Staubsammelfilter nach mindestens einem der Ansprüche 1 bis 18, **dadurch gekennzeichnet, dass** das Adsorptionsmaterial chemisch und/oder physikalisch an das Trägermaterial gebunden ist.

20. Staubsammelfilter nach mindestens einem der Ansprüche 1 bis 19, **dadurch gekennzeichnet, dass** das Adsorptionsmaterial an ein elektrostatisch geladenes Trägermaterial gebunden ist.

21. Staubsammelfilter nach mindestens einem der Ansprüche 1 bis 20, **dadurch gekennzeichnet, dass** das Adsorptionsmaterial pulverförmig ist und eine mittlere Korngröße von 1 bis 100 µm aufweist.

22. Staubsammelfilter nach mindestens einem der Ansprüche 1 bis 21, **dadurch gekennzeichnet, dass** das Trägermaterial Fasern sind, die aus Chemiefasern und/oder Naturfasern ausgewählt sind.

23. Staubsammelfilter nach Anspruch 22, **dadurch gekennzeichnet,**
**dass** die Fasern antibakteriell ausgerüstet sind.

24. Staubsammelfilter nach Anspruch 22 oder 23, **dadurch gekennzeichnet,**
**dass** die Chemiefasern cellulosische Fasern, wie Viskose und/oder synthetische Fasern sind.

25. Staubsammelfilter nach Anspruch 24, **dadurch gekennzeichnet,**
**dass** die synthetischen Fasern ausgewählt sind aus Fasern aus Polyolefinen, Polyester, Polyamiden, Polyacrylnitril und/oder Polyvinylalkohol.

26. Staubsammelfilter nach Anspruch 22 oder 23, **dadurch gekennzeichnet,**
**dass** die Naturfasern ausgewählt sind aus Cellulose, Holzfaserstoffe, Kapok, Flachs, Jute, Manilahanf, Kokos, Wolle, Baumwolle, Kenaf, Abaca, Maulbeerbast und/oder Fluffpulp.

27. Staubsammelfilter nach mindestens einem der Ansprüche 22 bis 26, **dadurch gekennzeichnet,**
**dass** die Fasern glatt, verzweigt, gekrimpt, hohl und/oder texturiert sind und/oder einen nicht kreisförmigen (z. B. trilobalen) Querschnitt aufweisen.

28. Staubsammelfilter nach mindestens einem der Ansprüche 22 bis 27, **dadurch gekennzeichnet,**
**dass** die Fasern eine mittlere Länge zwischen 0,3 mm und 100 mm, bevorzugt zwischen 0,5 und 70 mm aufweisen.

29. Staubsammelfilter nach Anspruch 28, **dadurch gekennzeichnet,**
**dass** die Fasern eine mittlere Länge von 1 bis 9,5 mm aufweisen.

30. Staubsammelfilter nach mindestens einem der Ansprüche 1 bis 29, **dadurch gekennzeichnet,**
**dass** das Trägermaterial Flocken sind, die aus Schaumstoffen, Vliesstoffen, Textilien, geschäumter Stärke, geschäumten Polyolefinen, sowie Folien und Reißfasern ausgewählt sind.

31. Staubsammelfilter nach Anspruch 30, **dadurch gekennzeichnet,**
**dass** die Flocken einen Durchmesser von 0,3 mm bis 30 mm, bevorzugt 0,5 bis 20 mm, aufweisen.

32. Staubsammelfilter nach Anspruch 31, **dadurch gekennzeichnet,**
**dass** die Flocken einen Durchmesser von 1 bis 9,5 mm aufweisen.

33. Staubsammelfilter nach mindestens einem der Ansprüche 1 bis 32, **dadurch gekennzeichnet, dass** das Trägermaterial Granulate sind, die ausgewählt sind aus makroporösen Polymeren.

34. Staubsammelfilter nach Anspruch 33, **dadurch gekennzeichnet, dass** die Partikelgröße der Granulate im Bereich von 0,2 bis 1,5 mm, bevorzugt von 0,3 bis 1,0 mm, liegt.

35. Staubsammelfilter nach Anspruch 33 oder 34, **dadurch gekennzeichnet, dass** die makroporösen Polymere aufgebaut sind aus Polystyrol, Acrylsäure und/oder deren Derivaten.

36. Staubsammelfilter nach mindestens einem der Ansprüche 33 bis 36, **dadurch gekennzeichnet, dass** die Oberfläche der makroporösen Polymere > 200 m²/g, bevorzugt > 350 m²/g, ist.

37. Staubsammelfilter nach mindestens einem der Ansprüche 33 bis 36, **dadurch gekennzeichnet, dass** die Porosität ≥ 0,4 ml/ml ist.

38. Staubsammelfilter nach mindestens einem der Ansprüche 1 bis 37, **dadurch gekennzeichnet,**
**dass** das Adsorbens in einer luftdurchlässigen Umhüllung eingeschlossen ist.

39. Staubsammelfilter nach Anspruch 38, **dadurch gekennzeichnet,**
**dass** die Umhüllung ein luftdurchlässiges Vlies ist.

40. Staubsammelfilter nach einem der Ansprüche 1 bis 39, **dadurch gekennzeichnet,**
**dass** im Staubsammelfilter pro 1000 cm³ Volumen 0,03 bis 5 g des Adsorbens enthalten sind.

41. Staubsammelfilter nach Anspruch 41, **dadurch gekennzeichnet,**
**dass** pro 1000 cm³ 0,3 bis 2 g Adsorbens enthalten sind.

42. Staubsammelfilter nach einem der Ansprüche 1 bis 41, **dadurch gekennzeichnet,**
**dass** das Adsorbens in einem in einer luftdurchlässigen Umhüllung aufweisenden Beutel im Staubsammelfilter vorliegt.

43. Staubsammelfilter nach Anspruch 42, **dadurch gekennzeichnet,**
**dass** das Adsorbens in einem Teilbereich der Innenfläche des Staubsammelfilters unter einer Abdeckung angeordnet ist.

44. Staubsammelfilter nach Anspruch 43, **dadurch gekennzeichnet,**
**dass** die Abdeckung eine Vliesschicht ist.

45. Staubsammelfilter nach Anspruch 43, **dadurch gekennzeichnet,**
**dass** das Adsorbens in einem Kissen enthalten ist, das auf einem Teilbereich der Innenfläche des Staubsammelfilters angeordnet ist.

46. Staubsammelfilter nach Anspruch 45, **dadurch gekennzeichnet,**
**dass** das Kissen aus mindestens einer Schicht eines Filterpapiers oder eines speziellen Vlieses besteht, wobei das auf der Oberfläche des Filterpapier angeordnete Adsorbens durch mindestens eine Vliesschicht abgedeckt ist.

47. Staubsammelfilter nach mindestens einem der Ansprüche 42 bis 46, **dadurch gekennzeichnet,**
**dass** das Umhüllungsmaterial des Beutels bzw. die Abdeckung aus einem unter Betriebsbedingungen zerstörbaren Material gebildet ist.

48. Staubsammelfilter nach mindestens einem der Ansprüche 1 bis 47, **dadurch gekennzeichnet,**
**dass** der Staubsammelfilter so dimensioniert und ausgelegt ist, dass er mit einem Volumenstrom von 10 cm³/h bis 400 m³/h betrieben werden kann.

49. Staubsammelfilter nach mindestens einem der Ansprüche 1 oder 40, **dadurch gekennzeichnet,**
**dass** das Filtermaterial des Staubsammelfilters ein ein- oder mehrschichtiges Papier und/oder Vliesmaterial ist.

50. Staubsammelfilter nach mindestens einem der Ansprüche 1 bis 49, **dadurch gekennzeichnet,**
**dass** er ein Staubsaugerbeutel ist.

51. Staubsammelfilter nach mindestens einem der Patentansprüche 1 bis 49, **dadurch gekennzeichnet, dass** er ein plissierter Filter oder Taschenfilter ist.

52. Verfahren zur Geruchsadsorbtion mit einem Staubsammelfilter, **dadurch gekennzeichnet,**
**dass** mit einem Staubsammelfilter nach einem der Ansprüche 1 bis 51 gearbeitet wird.

53. Verfahren nach Anspruch 52, **dadurch gekennzeichnet,**
**dass** 0,2 bis 5 g Adsorbens pro 1000 cm³ Staubsammelfilter verwendet wird.

54. Verfahren nach Anspruch 52 oder 53, **dadurch gekennzeichnet,**
**dass** vor Beginn eines erstmaligen Saugvorgangs oder bei Beginn des Saugvorgangs das Adsorbens in den Staubsammelfilter eingebracht wird.

55. Verfahren nach mindestens einem der Ansprüche 52 oder 54, **dadurch gekennzeichnet,**
**dass** das Adsorbens in einer Umhüllung vorliegt und vor Beginn eines erstmaligen Saugvorgangs oder bei Beginn des Saugvorgangs in den Staubsammelfilter eingebracht wird.

56. Verfahren nach Anspruch 55, **dadurch gekennzeichnet,**
**dass** die Umhüllung so gewählt wird, dass sie unter dem gegebenen Volumenstrom zerstört wird.

57. Verfahren nach mindestens einem der Ansprüche 52 bis 56, **dadurch gekennzeichnet,**
**dass** es sich um ein Verfahren zum Staubsaugen mit einem Bodenstaubsauger oder einem Handstaubsauger handelt.

## Claims

1. Dust-collecting filter formed from an air-permeable filter material, an adsorbing agent being contained in loose form in the dust-collecting filter, **characterised in that** the adsorbing agent comprises fibres, flakes and/or granulate as a supporting material onto which a powdery adsorption material is applied superficially.

2. Dust-collecting filter according to claim 1, **characterised in that** the adsorption material is applied in an amount of 1 to 50 wt-% of the supporting material.

3. Dust-collecting filter according to claim 2, **characterised in that** 7 to 25 wt-% are applied.

4. Dust-collecting filter according to at least one of claims 1 to 3, **characterised in that** the adsorption material is selected from active charcoal, impregnated active charcoal, functionalised carbon, hydrophobic zeolites, hydrophobic, porous polymers, bentonites and/or crystalline organometallic complexes.

5. Dust-collecting filter according to claim 4, **characterised in that** the functionalised carbon is an aromatic carbon skeleton with functional groups.

6. Dust-collecting filter according to claim 4, **characterised in that** the active charcoal is coconut shell, wood, rock or bamboo charcoal.

7. Dust-collecting filter according to claim 4 or 5, **characterised in that** the active charcoal is impregnated with acid or basic chemicals and/or with silver salts.

8. Dust-collecting filter according to claim 4, **characterised in that** the zeolites have micropores of a pore size > 5 Å.

9. Dust-collecting filter according to claim 8, **characterised in that** the pore size of the micropores is > 6.5 Å.

10. Dust-collecting filter according to claim 8 or 9, **characterised in that** the specific surface of the zeolites is > 400 m²/g.

11. Dust-collecting filter according to at least one of claims 8 to 10, **characterised in that** the zeolites have a modulus or a ratio of SiO₂/Al₂O₃> 200, preferably > 300.

12. Dust-collecting filter according to at least one of claims 8 to 11, **characterised in that** the particle size of the zeolites is in the range between 2 and 30 µm.

13. Dust-collecting filter according to claim 4, **characterised in that** the porous polymers have micropores of 6 to 20 Å, mesopores of 20 to 500 Å and macropores > 500 Å.

14. Dust-collecting filter according to claim 4 or 13, **characterised in that** the average pore diameter is between 3 and 300 Å.

15. Dust-collecting filter according to claim 4, 13 or 14, **characterised in that** the particle size of the porous polymers is in the range between 1 and 500 µm, preferably between 1 and 200 µm.

16. Dust-collecting filter according to at least one of claims 4 or 13 to 15, **characterised in that** the pore volume is ≥ 0.4 cm³/g.

17. Dust-collecting filter according to at least one of claims 4 or 13 to 15, **characterised in that** the porous polymers are hydrophobic.

18. Dust-collecting filter according to at least one of claims 4 or 13 to 17, **characterised in that** the porous polymers are constructed from styrene, acrylic acid and/or their derivatives.

19. Dust-collecting filter according to at least one of claims 1 to 18, **characterised in that** the adsorption material is chemically and/or physically bound to the supporting material.

20. Dust-collecting filter according to at least one of claims 1 to 19, **characterised in that** the adsorption material is bound to an electrostatically charged supporting material.

21. Dust-collecting filter according to at least one of claims 1 to 20, **characterised in that** the adsorption material is powdery and has a mean particle size of 1 to 100 µm.

22. Dust-collecting filter according to at least one of claims 1 to 21, **characterised in that** the supporting material comprises fibres which are selected from chemical fibres and/or natural fibres.

23. Dust-collecting filter according to claim 22, **characterised in that** the fibres are rendered antibacterial.

24. Dust-collecting filter according to claim 22 or 23, **characterised in that** the chemical fibres are cellulose fibres such as viscose and/or synthetic fibres.

25. Dust-collecting filter according to claim 24, **characterised in that** the synthetic fibres are selected from fibres formed from polyolefins, polyester, polyamides, polyacrylonitrile and/or polyvinyl alcohol.

26. Dust-collecting filter according to claim 22 or 23, **characterised in that** the natural fibres are selected from cellulose, wood fibre materials, kapok, flax, jute, Manila hemp, coco, wool, cotton, Kenaf, abaca, mulberry bast and/or fluff pulp.

27. Dust-collecting filter according to at least one of claims 22 to 26, **characterised in that** the fibres are smooth, branched, crimped, hollow and/or textured and have a non-circular (e.g. trilobal) cross-section.

28. Dust-collecting filter according to at least one of claims 22 to 27, **characterised in that** the fibres have a mean length of between 0.3 mm and 100 mm, preferably between 0.5 and 70 mm.

29. Dust-collecting filter according to claim 28, **characterised in that** the fibres have a mean length of 1 to 9.5 mm.

30. Dust-collecting filter according to at least one of claims 1 to 29, **characterised in that** the supporting material comprises flakes which are selected from cellular plastics, non-wovens, textiles, foamed starch, foamed polyolefins, as well as films and recovered fibres.

31. Dust-collecting filter according to claim 30, **characterised in that** the flakes have a diameter of 0.3 mm to 30 mm, preferably 0.5 to 20 mm.

32. Dust-collecting filter according to claim 31, **characterised in that** the flakes have a diameter of 1 to 9.5 mm.

33. Dust-collecting filter according to at least one of claims 1 to 32, **characterised in that** the supporting material comprises granulates which are selected from macroporous polymers.

34. Dust-collecting filter according to claim 33, **characterised in that** the particle size of the granulates is in the range between 0.2 and 1.5 mm, preferably between 0.3 and 1.0 mm.

35. Dust-collecting filter according to claim 33 or 34, **characterised in that** the macroporous polymers are constructed from polystyrene, acrylic acid and/or their derivatives.

36. Dust-collecting filter according to at least one of claims 33 to 36, **characterised in that** the surface of the macroporous polymers is > 200 m²/g, preferably > 350 m²/g.

37. Dust-collecting filter according to at least one of claims 33 to 36, **characterised in that** the porosity ≥ 0.4 ml/ml.

38. Dust-collecting filter according to at least one of claims 1 to 37, **characterised in that** the adsorbing agent is enclosed in an air-permeable wrapper.

39. Dust-collecting filter according to claim 38, **characterised in that** the wrapper is an air-permeable non-woven.

40. Dust-collecting filter according to one of claims 1 to 39, **characterised in that** 0.03 to 5 g of the adsorbing agent per 1000 cm³ are contained in the dust-collecting filter.

41. Dust-collecting filter according to claim 40, **characterised in that** 0.3 to 2 g adsorbing agent are contained per 1000 cm³.

42. Dust-collecting filter according to one of claims 1 to 41, **characterised in that** the adsorbing agent is present in a bag, which has an air-permeable wrapper, in the dust-collecting filter.

43. Dust-collecting filter according to claim 42, **characterised in that** the adsorbing agent is arranged under a covering in part of the inner surface of the dust-collecting filter.

44. Dust-collecting filter according to claim 43, **characterised in that** the covering is a non-woven layer.

45. Dust-collecting filter according to claim 43, **characterised in that** the adsorbing agent is contained in a pad which is arranged on part of the inner surface of the dust-collecting filter.

46. Dust-collecting filter according to claim 45, **characterised in that** the pad comprises at least one layer of a filter paper or of a special non-woven, the adsorbing agent arranged on the surface of the filter paper being covered by at least one non-woven layer.

47. Dust-collecting filter according to at least one of claims 42 to 46, **characterised in that** the wrapper material of the bag or the covering is formed from a material which can be destroyed under operating conditions.

48. Dust-collecting filter according to at least one of claims 1 to 47, **characterised in that** the dust-collecting filter is of such dimensions and design that it can be operated with a volume flow rate of 10 cm³/h to 400 m³/h.

49. Dust-collecting filter according to at least one of claims 1 or 40, **characterised in that** the filter material of the dust-collecting filter is a single-layer or multilayer paper and/or non-woven material.

50. Dust-collecting filter according to at least one of claims 1 to 49, **characterised in that** it is a vacuum-cleaner bag.

51. Dust-collecting filter according to at least one of patent claims 1 to 49, **characterised in that** it is a pleated filter or bag filter.

52. Method for adsorbing odours with a dust-collecting filter, **characterised in that** a dust-collecting filter according to one of claims 1 to 51 is used for it.

53. Method according to claim 52, **characterised in that** 0.2 to 5 g adsorbing agent are used per 1000 cm³ dust-collecting filter.

54. Method according to claim 52 or 53, **characterised in that** the adsorbing agent is introduced into the dust-collecting filter before the start of a first suction process or at the start of the suction process.

55. Method according to at least one of claims 52 or 54, **characterised in that** the adsorbing agent is present in a wrapper and is introduced into the dust-collecting filter before the start of a first suction process or at the start of the suction process.

56. Method according to claim 55, **characterised in that** the wrapper is so selected that it is destroyed at the given volume flow rate.

57. Method according to at least one of claims 52 to 56, **characterised in that** this is a method for vacuum-cleaning using a cylinder vacuum-cleaner or an upright vacuum-cleaner.

## Revendications

1. Filtre collecteur de poussière constitué d'un matériau de filtre pouvant laisser passer l'air, le filtre collecteur de poussière contenant un agent adsorbant en vrac, **caractérisé en ce que** l'agent adsorbant est constitué de fibres, de flocons et/ou de granulés faisant fonction de matériau porteur, sur lesquels un matériau d'adsorption pulvérulent est appliqué en surface.

2. Filtre collecteur de poussière selon la revendication 1, **caractérisé en ce que** le matériau d'adsorption est appliqué en une quantité allant de 1 à 50 % en poids du matériau porteur.

3. Filtre collecteur de poussière selon la revendication 2, **caractérisé en ce que** de 7 à 25 % en poids sont appliqués.

4. Filtre collecteur de poussière selon au moins une des revendications 1 à 3, **caractérisé en ce que** le matériau d'adsorption est choisi parmi du charbon actif, du charbon actif imprégné, du carbone fonctionnalisé, de la zéolite hydrophobe, des polymères poreux hydrophobes, de la bentonite et/ou des complexes organométalliques cristallins.

5. Filtre collecteur de poussière selon la revendication 4, **caractérisé en ce que** le carbone fonctionnalisé est un squelette en carbone aromatique ayant des groupes fonctionnels.

6. Filtre collecteur de poussière selon la revendication 4, **caractérisé en ce que** le charbon actif est des coques de noix de coco, du charbon de bois, de la houille ou du charbon de bambou.

7. Filtre collecteur de poussière selon la revendication 4 ou 5, **caractérisé en ce que** le charbon actif est imprégné de produits chimiques acides ou basiques et/ou de sels d'argent.

8. Filtre collecteur de poussière selon la revendication 4, **caractérisé en ce que** les micropores de zéolite ont une taille de pore > 5Å.

9. Filtre collecteur de poussière selon la revendication 8, **caractérisé en ce que** la taille de pore des micropores est > 6,5Å.

10. Filtre collecteur de poussière selon la revendication 8 ou 9, **caractérisé en ce que** la surface spécifique de la zéolite est > 400 m²/g.

11. Filtre collecteur de poussière selon au moins une des revendications 8 à 10, **caractérisé en ce que** la zéolite présente un module ou un comportement de SiO₂/Al₂O₃ > 200, de préférence > 300.

12. Filtre collecteur de poussière selon au moins une des revendications 8 à 11, **caractérisé en ce que** la taille de particule de la zéolite se situe dans une gamme de 2 à 30 µm.

13. Filtre collecteur de poussière selon la revendication 4, **caractérisé en ce que** les micropores de polymères poreux présentent une taille de 6 à 20 Å, les mésopores une taille de 20 à 500 Å et les macropores une taille > 500 Å.

14. Filtre collecteur de poussière selon la revendication 4 ou 13, **caractérisé en ce que** le diamètre moyen de pore se situe entre 3 et 300 Å.

15. Filtre collecteur de poussière selon la revendication 4, 13 ou 14, **caractérisé en ce que** la taille de particule des polymères poreux se situe dans la gamme de 1 à 500 µm, de préférence de 1 à 200 µm.

16. Filtre collecteur de poussière selon au moins une des revendications 4 ou 13 à 15, **caractérisé en ce que** le volume de pore est ≥ 0,4 cm³/g.

17. Filtre collecteur de poussière selon au moins une des revendications 4 ou 13 à 15, **caractérisé en ce que** les polymères poreux sont hydrophobes.

18. Filtre collecteur de poussière selon au moins une des revendications 4 ou 13 à 17, **caractérisé en ce que** les polymères poreux sont constitués de styrène, d'acide acrylique et/ou de leurs dérivés.

19. Filtre collecteur de poussière selon au moins une des revendications 1 à 18, **caractérisé en ce que** le matériau d'adsorption est lié de manière chimique et/ou de manière physique au matériau porteur.

20. Filtre collecteur de poussière selon au moins une des revendications 1 à 19, **caractérisé en ce que** le matériau d'adsorption est lié à un matériau porteur chargé électrostatiquement.

21. Filtre collecteur de poussière selon au moins une des revendications 1 à 20, **caractérisé en ce que** le matériau d'adsorption est pulvérulent et présente une taille moyenne de grain allant de 1 à 100 µm.

22. Filtre collecteur de poussière selon au moins une des revendications 1 à 21, **caractérisé en ce que** le matériau porteur est constitué de fibres, qui sont choisies parmi des fibres synthétiques et/ou des fibres naturelles.

23. Filtre collecteur de poussière selon la revendication 22, **caractérisé en ce que** les fibres ont des propriétés antibactériennes.

24. Filtre collecteur de poussière selon la revendication 22 ou 23, **caractérisé en ce que** les fibres synthétiques sont des fibres cellulosiques, telles que des fibres de viscose et/ou des fibres synthétiques.

25. Filtre collecteur de poussière selon la revendication 24, **caractérisé en ce que** les fibres synthétiques sont choisies parmi des fibres de polyoléfines, de polyester, de polyamides, de polyacrylonitrile et/ou de polyalcool vinylique.

26. Filtre collecteur de poussière selon la revendication 22 ou 23, **caractérisé en ce que** les fibres naturelles sont choisies parmi la cellulose, les tissus en fibres de bois, le kapok, le lin, le jute, le chanvre de Manille, la noix de coco, la laine, le coton, le kénaf, l'abaca, le raphia de mûres et/ou la pulpe de cellulose.

27. Filtre collecteur de poussière selon au moins une des revendications 22 à 26, **caractérisé en ce que** les fibres sont plates, ramifiées, rétrécies, creuses et/ou texturées et/ou ne présentent pas de coupe transversale circulaire (par exemple trilobale).

28. Filtre collecteur de poussière selon au moins une des revendications 22 à 27, **caractérisé en ce que** les fibres présentent une longueur moyenne comprise entre 0,3 mm et 100 mm, de préférence comprise entre 0,5 et 70 mm.

29. Filtre collecteur de poussière selon la revendication 28, **caractérisé en ce que** les fibres présentent une longueur moyenne de 1 à 9,5 mm.

30. Filtre collecteur de poussière selon au moins une des revendications 1 à 29, **caractérisé en ce que** le matériau porteur est constitué flocons, qui sont choisis parmi des mousses, des non tissés, des textiles, des mousses d'amidon, des mousses de polyoléfines, de même que des films et des fibres de riz.

31. Filtre collecteur de poussière selon la revendication 30, **caractérisé en ce que** les flocons présentent un diamètre allant de 0,3 mm à 30 mm, de préférence allant de 0,5 à 20 mm.

32. Filtre collecteur de poussière selon la revendication 31, **caractérisé en ce que** les flocons présentent un diamètre allant de 1 à 9,5 mm.

33. Filtre collecteur de poussière selon au moins une des revendications 1 à 32, **caractérisé en ce que** le matériau porteur est constitué granulés, qui sont choisis parmi des polymères macroporeux.

34. Filtre collecteur de poussière selon la revendication 33, **caractérisé en ce que** la taille de particule des granulés se situe dans la gamme de 0,2 à 1,5 mm, de préférence de 0,3 à 1,0 mm.

35. Filtre collecteur de particules selon la revendication 33 ou 34, **caractérisé en ce que** les polymères macroporeux sont constitués de polystyrène, d'acide acrylique et/ou de leurs dérivés.

36. Filtre collecteur de poussière selon au moins une des revendications 33 à 36, **caractérisé en ce que** la surface des polymères macroporeux est > 200 m²/g, de préférence >350 ₘ²_{/}g.

37. Filtre collecteur de poussière selon au moins une des revendications 33 à 36, **caractérisé en ce que** la porosité est ≥ 0,4 ml/ml.

38. Filtre collecteur de poussière selon au moins une des revendications 1 à 37, **caractérisé en ce que** l'agent adsorbant est enfermé dans une enveloppe laissant passer l'air.

39. Filtre collecteur de poussière selon la revendication 38, **caractérisé en ce que** l'enveloppe est un non tissé laissant passer l'air.

40. Filtre collecteur de poussière selon l'une des revendications 1 à 39, **caractérisé en ce que**, dans le filtre collecteur de poussière, 0,03 à 5 g de l'agent adsorbant sont contenus par volume de 1 000 cm³.

41. Filtre collecteur de poussière selon la revendication 41, **caractérisé en ce que** 0,3 à 2 g d'agent adsorbant sont contenus par 1 000 cm³.

42. Filtre collecteur de poussière selon l'une des revendications 1 à 41, **caractérisé en ce que** l'agent adsorbant dans un sac présentant une enveloppe laissant passer l'air se trouve dans un filtre collecteur de poussière.

43. Filtre collecteur de poussière selon la revendication 42, **caractérisé en ce que** l'agent adsorbant est disposé dans une partie de la surface interne du film collecteur de poussière sous un cache.

44. Filtre collecteur de poussière selon la revendication 43, **caractérisé en ce que** le cache est une couche de non tissé.

45. Filtre collecteur de poussière selon la revendication 43, **caractérisé en ce que** l'agent adsorbant est contenu dans un coussin, qui est disposé sur une partie de la surface interne du filtre collecteur de poussière.

46. Filtre collecteur de poussière selon la revendication 45, **caractérisé en ce que** le coussin est constitué d'au moins une couche de papier-filtre ou de non tissé spécifique, l'agent adsorbant disposé sur la surface du papier-filtre étant recouvert d'au moins une couche de non tissé.

47. Filtre collecteur de poussière selon au moins une des revendications 42 à 46, **caractérisé en ce que** le matériau d'enveloppe du sac respectivement du cache est formé d'un matériau destructible dans les conditions de fonctionnement.

48. Filtre collecteur de poussière selon au moins une des revendications 1 à 47, **caractérisé en ce que** le filtre collecteur de poussière est dimensionné et garni de sorte qu'il peut être exploité avec un débit volumique de 10 cm³/h à 400 m³/h.

49. Filtre collecteur de poussière selon au moins une des revendications 1 ou 40, **caractérisé en ce que** le matériau de filtre du filtre collecteur de poussière est un papier mono ou multicouche et/ou un matériau non tissé.

50. Filtre collecteur de poussière selon au moins une des revendications 1 à 49, **caractérisé en ce qu'**il est un sac pour aspirateur.

51. Filtre collecteur de poussière selon au moins une des revendications 1 à 49, **caractérisé en ce qu'**il est un filtre ou un filtre de type poche plissé.

52. Procédé pour l'adsorption des odeurs à l'aide d'un filtre collecteur de poussière, **caractérisé en ce qu'**il est mis en oeuvre à l'aide d'un filtre collecteur de poussière selon l'une des revendications 1 à 51.

53. Procédé selon la revendication 52, **caractérisé en ce que** de 0,2 à 5 g d'agent adsorbant pour 1 000 cm³ de filtre collecteur de poussière sont utilisés.

54. Procédé selon la revendication 52 ou 53, **caractérisé en ce que** l'adsorbant est introduit dans le filtre collecteur de poussière avant le début de la première aspiration ou au début de l'aspiration.

55. Procédé selon au moins une des revendications 52 ou 54, **caractérisé en ce que** l'adsorbant se trouve dans un emballage et est introduit dans le filtre collecteur de poussière avant le début de la première aspiration ou au début de l'aspiration.

56. Procédé selon la revendication 55, **caractérisé en ce que** l'emballage est choisi de sorte qu'il est détruit au débit volumique donné.

57. Procédé selon au moins une des revendications 52 à 56, **caractérisé en ce qu'**il s'agit d'un procédé pour l'aspiration de poussière à l'aide d'un aspirateur pour le sol ou d'un aspirateur portable.
